(19) **Europäisches Patentamt / European Patent Office / Office européen des brevets**

(11) **EP 4 555 947 A1**

(12) **EUROPEAN PATENT APPLICATION**
published in accordance with Art. 153(4) EPC

(43) Date of publication:
**21.05.2025 Bulletin 2025/21**

(21) Application number: **23839997.6**

(22) Date of filing: **13.07.2023**

(51) International Patent Classification (IPC):
*A61B 17/221* (2006.01)     *A61B 34/30* (2016.01)
*A61B 17/22* (2006.01)     *A61B 17/00* (2006.01)

(52) Cooperative Patent Classification (CPC):
**A61B 17/00; A61B 17/22; A61B 17/221;
A61B 34/30**

(86) International application number:
**PCT/KR2023/010044**

(87) International publication number:
**WO 2024/014906 (18.01.2024 Gazette 2024/03)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **13.07.2022 KR 20220086608**

(71) Applicant: **ROEN Surgical, Inc.
Daejeon 34051 (KR)**

(72) Inventors:
• **KONG, Duk-Yoo
  Daejeon 34051 (KR)**
• **KWON, Joon-Yeong
  Daejeon 34051 (KR)**
• **KIM, Jae-Chul
  Daejeon 34051 (KR)**
• **KIM, Joon-Yeong
  Daejeon 34051 (KR)**

• **KIM, Joon-Hwan
  Daejeon 34051 (KR)**
• **SA ROSA, Tuani De
  Daejeon 34051 (KR)**
• **BAEK, Hyun-Woo
  Daejeon 34051 (KR)**
• **SEO, Hyeon Se
  Daejeon 34051 (KR)**
• **SONG, Kyu-Seob
  Daejeon 34051 (KR)**
• **YANG, Un-Je
  Daejeon 34051 (KR)**
• **LEE, Dong-Ho
  Daejeon 34051 (KR)**
• **JANG, Jinhyeok
  Daejeon 34051 (KR)**
• **JEON, Jiun
  Daejeon 34051 (KR)**

(74) Representative: **Stolmár & Partner
Patentanwälte PartG mbB
Blumenstraße 17
80331 München (DE)**

(54) **SYSTEM AND METHOD FOR PROVIDING GUIDANCE FOR CALCULUS REMOVAL**

(57)     A system and method for providing guidance according to an embodiment comprises a slave device, a processor, and a master device. The slave device includes: a guide tube, which can be inserted into the ureter, for calculus removal; a wire that can move inside the guide tube along the longitudinal axis of the guide tube; a driving unit for moving the wire; a medical tool that is connected to the distal end portion of the wire and exposed to the outside of the guide tube; and a camera that captures medical images at the distal end portion of the guide tube. The processor determines, on the basis of the estimated distance between the medical tool and the camera at the distal end portion of the guide tube, the size of a guidance graphic representation indicating the calculus size that can be gripped by a basket. The master device displays the guidance graphic representation having the determined size by overlaying the guidance graphic representation on the medical image captured by the camera and received from the slave device.

EP 4 555 947 A1

[FIG. 1]

## Description

### Technical Field

Hereinafter, there is proposed a technique that provides guidance for calculus removal using a basket and laser fiber.

### Background Art

The term urinary calculus refers to a solid mass (e.g., a stone) that forms in the urinary tract, which is the passage that produces and excretes urine. When the concentration of minerals (e.g., calcium, oxalic acid, phosphoric acid, uric acid, etc.) dissolved in urine increases, they can no longer dissolve in the urine and turn into crystals. These crystals grow and aggregate to form a calculus. Most urinary calculi are formed in the kidneys and are found in the renal pelvis, ureters, bladder, and urethra. Pain from urinary calculi varies depending on the shape, size, and location of the calculus. All types of urinary calculi are accompanied by hematuria, which is blood in the urine, and among these, kidney calculi have no symptoms at all or only mild indigestion. In terms of pain, when a ureteral calculus is stuck in the upper ureter, pain spreads from the side to the back, and when it is stuck in the lower ureter, pain spreads from the lower abdomen to the groin, thigh, and perineum. Bladder calculi can cause symptoms such as lower abdominal discomfort, frequent urination, and unpleasant urination.

Urinary calculi can be diagnosed by performing a urine chemical analysis test, X-ray examination, CT, urography, or ultrasound examination. Additionally, urinary calculi can be removed using extracorporeal shock wave lithotripsy (ESWL), retrograde intrarenal surgery (RIRS), percutaneous nephrolithotripsy (PCNL), or open or laparoscopic surgery.

The above description has been possessed or acquired by the inventor(s) in the course of conceiving the disclosure of the present application and is not necessarily an art publicly known before the present application is filed.

### Disclosure

### Technical Problem

One objective of the present disclosure is to provide a system and method for providing guidance for calculus removal, the system and method being capable of providing a guidance graphic representation that is displayed overlaid on a medical image.

Another objective of the present disclosure is to provide a system and method for providing guidance for calculus removal, the system and method being capable of providing a guidance graphic representation when using a basket and a laser fiber among medical tools.

Another objective of the present disclosure is to provide a system and method for providing guidance for calculus removal, the system and method being capable of providing a guidance graphic representation in different sizes according to a changing estimated distance.

Another objective of the present disclosure is to provide a system and method for providing guidance for calculus removal, the system and method being capable of estimating a distance between a medical tool and a camera at a distal end of a guide tube by simultaneously using image-based distance estimation and encoder-based distance estimation.

However, the objectives of the present disclosure are not limited to those described above, and other objectives will be apparent from the following detailed description.

### Technical Solution

One aspect of present disclosure provides a system for providing guidance for calculus removal, the system including: a slave device including a guide tube that is configured to be inserted into a ureter for calculus removal, a wire that is configured to be movable along a longitudinal axis of the guide tube within the guide tube, a driving unit that is configured to move the wire, a medical tool that is connected to a distal end portion of the wire to be exposed out of the guide tube and includes a basket or laser fiber, and a camera that is configured to capture a medical image at a distal end portion of the guide tube; a processor configured to determine a size of a guidance graphic representation indicating a calculus size that is extractable by the basket on the basis of an estimated distance between the medical tool and the camera at the distal end portion of the guide tube; and a master device configured to display the guidance graphic representation having the determined size by overlaying the guidance graphic representation on the medical image captured by the camera and received from the slave device.

The processor may infer an image-based distance between the medical tool and the camera at the distal end portion of the guide tube from the captured medical image, the processor may estimate an encoder-based distance between the medical tool and the camera at the distal end portion of the guide tube from information about an encoder of the driving unit, and the processor may determine the estimated distance on the basis of the image-based distance and the encoder-based distance.

When the medical tool is the basket, the processor may estimate geometric information about a shaft from a shaft region of the basket from the captured medical image using a machine learning model, for example, an object detection or instance segmentation algorithm, and the processor may infer the image-based distance from the estimated geometric information on the basis of the machine learning model.

The geometric information about the shaft may include at least one of an area occupied by the shaft region of the basket on the medical image, a diameter of an estimated circle corresponding to a distal end portion of the shaft, a radius of the estimated circle corresponding to the distal end portion of the shaft, a length from a boundary of the medical image to the distal end portion of the shaft, a length from a center of the medical image to the distal end portion of the shaft, and an inclination of the shaft.

The processor may infer the image-based distance by inputting the medical image into another machine learning model.

The processor may determine the estimated distance by applying a bias compensation value determined on the basis of the image-based distance and the encoder-based distance to the encoder-based distance.

The bias compensation value may have a value that converges over time after calculation of the estimated distance is started.

The processor may reduce the size of the guidance graphic representation when the estimated distance increases, and the processor may increase the size of the guidance graphic representation when the estimated distance decreases.

When the medical tool is the laser fiber, the processor may sense a moment when a distal end portion of the laser fiber makes contact with a calculus, the processor may always display a guidance circle according to a distance of the laser fiber and allow a surgeon to directly bring the laser fiber into contact with the calculus and compare a size of the guidance circle with the calculus, the processor may capture and store a target image at a time point where the distal end portion of the laser fiber makes contact with the calculus, and the processor may output the medical image to a display of the master device simultaneously with the target image.

The processor may receive an input from a user to select a point on the target image and, in response thereto, output the guidance graphic representation on the target image on the basis of the selected point.

The processor may detect a calculus region on the target image and output the guidance graphic representation on the target image on the basis of a reference point in the detected calculus region.

The guidance graphic representation may be expressed as one of circular, oval, square, polygonal, and closed curve shapes.

The size of the guidance graphic representation may be defined on the basis of a size of a sheath that guides the guide tube.

A boundary of the guidance graphic representation may have a thickness. The processor may reduce the thickness of the boundary of the guidance graphic representation when reliability of the estimated distance increases over time.

Another aspect of the present disclosure provides a method of providing guidance by a processor, the method including: obtaining a medical image captured by including a medical tool at a distal end portion of a guide tube inserted into a ureter; inferring an image-based distance between the medical tool and a camera at the distal end portion of the guide tube from the captured medical image; obtaining information about an encoder of a driving unit that moves a wire within the guide tube; estimating an encoder-based distance between the medical tool and the camera at the distal end portion of the guide tube from the information about the encoder; determining an estimated distance between the medical tool and the camera at the distal end portion of the guide tube on the basis of the image-based distance and the encoder-based distance; and displaying a guidance graphic representation of a size determined on the basis of the estimated distance by overlaying the guidance graphic representation on the medical image.

The inferring of the image-based distance may include: when the medical tool is a basket, estimating geometric information about a shaft from a shaft region of the medical tool from the captured medical image; and inferring the image-based distance from the estimated geometric information on the basis of a machine learning model, for example, an object detection or instance segmentation algorithm. When the medical tool is a laser fiber, geometric information about the laser fiber may include a pixel distance between a distal end portion of the laser fiber on the medical image and a center of a screen, a length from a boundary of the medical image to the distal end portion of the laser fiber, an area occupied by a laser fiber region, and a length of the distal end portion of the laser fiber, and the remaining steps may the same as for the basket.

The determining of the estimated distance may include: determining the estimated distance by applying a bias compensation value determined on the basis of the image-based distance and the encoder-based distance to the encoder-based distance. The bias compensation value may have a value that converges over time after calculation of the estimated distance is started.

The displaying of the guidance graphic representation by overlaying the guidance graphic representation on the medical image may further include: adjusting the size of the guidance graphic representation on the basis of a changing estimated distance between the medical tool and the camera at the distal end portion of the guide tube. The size of the guidance graphic representation may decrease as the estimated distance increases and may increase as the estimated distance decreases.

The method may further include: after the displaying of the guidance graphic representation by overlaying the guidance graphic representation on the medical image, sensing an external input of a user and changing a position of the guidance on the medical image in response to the external input of the user.

**Advantageous Effects**

According to a system and method for providing guidance for calculus removal, by providing a guidance graphic representation that is displayed overlaid on a medical image, it is possible to achieve calculus removal without damage to an internal organ, without requiring complicated calculations to determine the size of a calculus.

According to the system and method for providing guidance for calculus removal, by providing a guidance graphic representation when using a basket and a laser fiber among medical tools, it is possible to enable a user to determine whether a calculus is of a size that is removable using the basket, and whether it is necessary to disintegrate the calculus using the laser fiber.

According to the system and method for providing guidance for calculus removal, by providing a guidance graphic representation of a specific actual size as a circle of a different size according to a changing estimated distance, it is possible to accurately determine whether a calculus is removable regardless of the estimated distance, thereby enabling safe treatment to be performed.

According to the system and method for providing guidance for calculus removal, by estimating a distance between a medical tool and a camera at a distal end of a guide tube with high accuracy by simultaneously using image-based distance estimation and encoder-based distance estimation, it is possible to provide a highly reliable guide graphic representation to a user.

**Description of Drawings**

FIG. 1 illustrates a system for providing guidance for calculus removal according to one embodiment.

FIG. 2 illustrates a driving mechanism of a slave device according to one embodiment.

FIG. 3 illustrates a guide tube, a camera, and a medical tool inserted into a ureter according to one embodiment.

FIG. 4 exemplarily illustrates a basket gripping a calculus through the system for providing guidance for calculus removal according to one embodiment.

FIG. 5 exemplarily illustrates a basket or laser fiber gripping a calculus to infer an image-based distance in the system for providing guidance for calculus removal according to one embodiment.

FIG. 6 exemplarily illustrates a graph of an image-based distance corresponding to geometric information used in the system for providing guidance for calculus removal according to one embodiment.

FIG. 7 exemplarily illustrates markers provided on a medical tool used in the system for providing guidance for calculus removal according to one embodiment.

FIG. 8 exemplarily illustrates a graph of relationship between an image-based distance, an encoder-based distance, and an estimated distance in the system for providing guidance for calculus removal according to one embodiment.

FIG. 9 exemplarily illustrates a function fitting method for deriving a graphic representation radius used in the system for providing guidance for calculus removal according to one embodiment.

FIG. 10 exemplarily illustrates a graph of a graphic representation radius corresponding to an estimated distance used in the system for providing guidance for calculus removal according to one embodiment.

FIG. 11 exemplarily illustrates a change in a guidance graphic representation that is expressed differently according to a changing estimated distance in the system for providing guidance for calculus removal according to one embodiment.

FIG. 12 exemplarily illustrates a screen of a display on which a guidance graphic representation is output in the system for providing guidance for calculus removal according to one embodiment.

FIG. 13 exemplarily illustrates types of guidance graphic representations in the system for providing guidance for calculus removal according to one embodiment.

FIG. 14 exemplarily illustrates a thickness of a boundary of a guidance graphic representation in the system for providing guidance for calculus removal according to one embodiment.

FIG. 15 exemplarily illustrates a graph of results of verifying a polynomial regression function used to infer an image-based distance in the system for providing guidance for calculus removal according to one embodiment.

FIG. 16 exemplarily illustrates graphs of a bias compensation value used to calculate an estimated distance according to the size and time of a calculus in the system for providing guidance for calculus removal according to one embodiment, and comparison results between a distance estimated using each image estimation method and a real distance.

FIG. 17 illustrates a flowchart of a method of providing guidance for calculus removal according to one embodiment.

## Best Mode

The following detailed structural or functional description is provided only for illustrative purposes of embodiments of the present disclosure and various alterations and modifications may be made to the embodiments. Here, the embodiments are not construed as limited to the disclosure and should be understood to include all changes, equivalents, and replacements within the idea and the technical scope of the disclosure.

It will be understood that, although the terms "first", "second", etc. may be used herein to describe various elements, these elements should not be limited by these terms. These terms are only used to distinguish one element from another element. For instance, a first element discussed below could be termed a second element. Similarly, the second element could also be termed the first element.

It will be understood that when an element is referred to as being "coupled" to another element, it can be directly coupled to or connected to the other element or intervening elements may be present therebetween.

As used herein, the singular forms are intended to include the plural forms as well, unless the context clearly indicates otherwise. It will be further understood that the terms "comprise", "include", etc., when used in this specification, specify the presence of stated features, integers, steps, operations, elements, components, and/or combinations thereof but do not preclude the presence or addition of one or more other features, integers, steps, operations, elements, components, and/or combinations thereof.

Unless otherwise defined, all terms including technical and scientific terms used herein have the same meaning as commonly understood by one of ordinary skill in the art to which the present disclosure belongs. It should be understood that the terms defined by the dictionary are identical with the meanings within the context of the related art, and they should not be ideally or excessively formally defined unless the context clearly dictates otherwise in this specification.

Hereinafter, embodiments of the present disclosure will be described in detail with reference to the accompanying drawings. When describing the embodiments with reference to the accompanying drawings, like reference numerals refer to like components and a repeated description related thereto will be omitted.

Among calculus removal methods, extracorporeal shock wave lithotripsy (ESWL) is a limited calculus removal method in that it is suitable only for calculi with a diameter of 1 cm or less and only for a small amount of calculi stuck in the ureter. Additionally, among calculus removal methods, percutaneous nephrolithotripsy (PCNL) has the problem that it cannot be used in patients with a tendency to bleed. On the other hand, retrograde intrarenal surgery (RIRS) has no major limitations in use, and has the advantage of being able to be performed on all patients who are pregnant or have a tendency to bleed and being able to remove a hard calculus at one time. Additionally, the RIRS has been reported to have a high success rate of up to 94%, with a complication rate of at least 0%. Even when complications occur at a low probability, the severity of the complications is low. As the scope of application of the RIRS expands along with the development of flexible ureteroscopy technology, various studies are being conducted.

Meanwhile, the RIRS may be performed by directly approaching the kidney with an endoscope, directly disintegrating a calculus with a laser fiber, and griping the calculus with a basket and withdrawing it from the body. In the RIRS method, the size of the calculus may be an important factor in the procedure. This is because when the basket is used to remove a too large calculus, the calculus may get caught in a sheath and cause damage to the ureter. Additionally, when using the laser fiber to disintegrate this large calculus to make it smaller, excessive disintegration may result in a large number of calculi to be removed, so it takes a long time to grip and discharge them one by one with the basket. On the contrary, when the laser fiber adequately disintegrates the large calculus, there may be a case where the large calculus remains as it is, so when the basket grips the calculus, there is still a risk of damaging the ureter. When the calculus is determined to be large after it is gripped by the basket, a medical tool may need to be replaced from the basket back to the laser fiber to disintegrate the calculus, which is cumbersome. Additionally, the calculus may be identified by displaying images captured by an endoscopic camera on a display, so it is not easy to determine the size of the calculus through the images displayed on the display, and accordingly, it is also not easy to determine whether the above-described problems may occur.

In order to solve the above-described problems, the present specification proposes a system and a method that provide guidance to determine whether a calculus is removable.

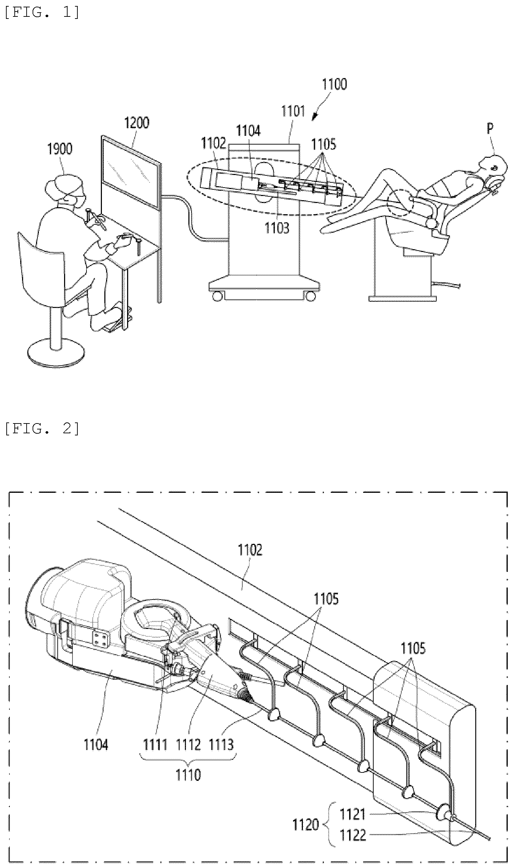

FIG. 1 illustrates a system for providing guidance for calculus removal according to one embodiment.

The system for providing guidance for calculus removal according to the embodiment of the present disclosure may display guidance on a medical image. The system for providing guidance may provide the medical image to a user 1900 (e.g., a practitioner or medical staff) during a kidney calculus removal procedure. The system for providing guidance may control a guide tube 1113, a camera 1114 (e.g., an endoscopic camera), and a medical tool 1107 for calculus removal on the basis of an input detected from the user 1900 through a manipulation part (e.g., a controller such as a touch screen or a master handle). For reference, the medical image in this specification may be an endoscopic image captured by an endoscopic camera.

The system for providing guidance may include a slave device 1100 and a master device 1200. The master device 1200 may be connected to the slave device 1100 via wires and/or wirelessly, and may exchange data. For example, the master device 1200 may transmit a control command based on the input from the user 1900 to the slave device 1100. The slave device 1100 may control movement and/or operation of at least one of the guide tube 1113, the camera 1114, and the medical tool 1107 in response to the control command. The slave device 1100 may transmit an operating status and a medical image to the master device 1200.

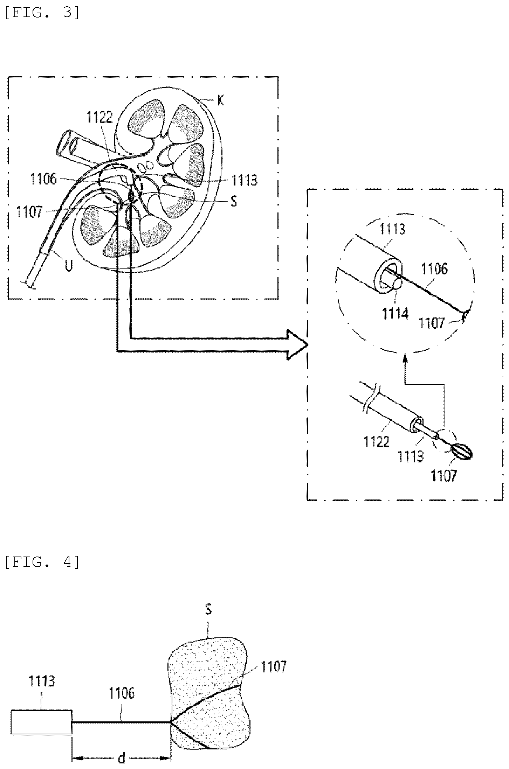

The slave device 1100 is a type of slave robot that is equipped with a medical tool and may operate the medical tool 1107 inserted into the body of a patient P under control of the user 1900 during the procedure. The slave device 1100 may include a support body 1101, a driving body 1102, a rail 1103, and a driving unit 1104. The slave device 1100 may control movement and/or operation of at least one of the guide tube 1113, the camera 1114, or and the medical tool 1107 by operating the driving unit 1104 under control of the master device 1200. For example, the slave device 1100 may apply at least one of movement (e.g., forward or backward movement), rotation, and bending to at least one of the guide tube 1113, the camera 1114, and the medical tool 1107. A more detailed structure of the slave device 1100 will be described later with reference to FIGS. 2 and 3.

The master device 1200 is a type of master console and may include a display, a controller, and a processor.

The display of the master device 1200 may provide medical information to the user 1900. For example, the display may visually output a medical image captured by the camera of the slave device 1100. Additionally, the display may output not only the real-time medical image described above, but also operation status information about the slave device 1100, function information, and procedure information as additional information to assist in the calculus removal procedure. The operation status information is information indicating a status related to operation of the slave device 1100, and may include, for example, a sheath length, a bending angle of the guide tube, and a rolling angle of the guide tube. The function information is information about a function provided through the master device 1200 and may include a recording function and a snapshot function. The procedure information may include, for example, information related to the calculus removal procedure, and may include information (e.g., an image) showing the shape and position of the guide tube inserted into the urethra.

The controller may receive an input from the user 1900. The master device 1200 may control the master device 1200 and/or the slave device 1100 by interpreting the input of the user 1900 received through the controller. For example, the controller may include at least one of a touchscreen, a touchpad, and a master handle. The touchscreen is a device integrated with the above-described display. Although it is mainly described herein that the display is a touch screen is mainly described, the present disclosure is not limited thereto. The master handle may receive a manipulation from the user 1900 for operation of the slave device 1100 (e.g., movement of the guide tube 1113, the camera 1114, or the medical tool 1107.

The processor may perform processing and interpretation of medical images and control of the slave device 1100. For example, the processor may generate a control command to control the slave device 1100 on the basis of interpretation of a manipulation of the user 1900 detected through the controller (e.g., the master handle). The processor may transmit the generated control command to the slave device 1100.

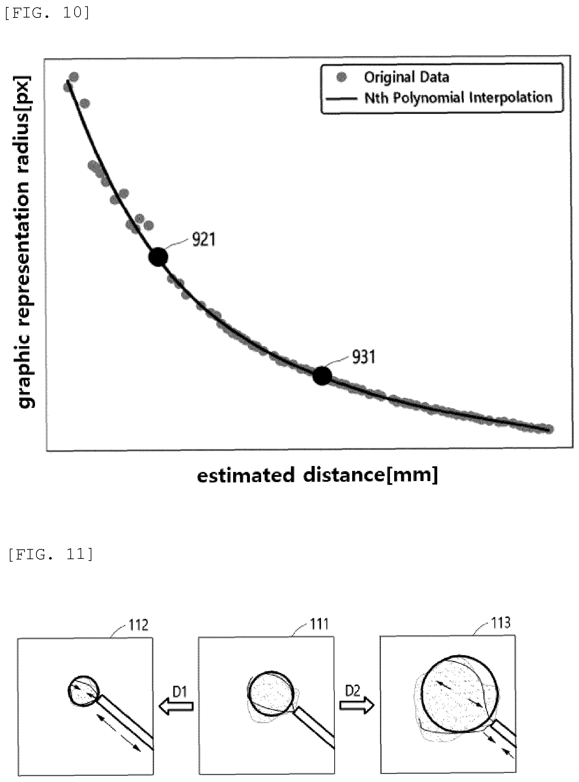

The processor may receive a medical image from the slave device 1100 and process or analyze the received medical image. For example, the processor may determine a guidance graphic representation to be displayed on the medical image. The processor may determine the size of the guidance graphic representation on the basis of an estimated distance between the medical tool and the camera at a distal end portion of the guide tube. The size of the guidance graphic representation may indicate a calculus size that can be extracted by a basket. The size at which the guidance graphic representation is displayed on the display will be described later with reference to FIG. 10. The master device 1200 may display the guidance graphic representation having the determined size by overlaying it on the medical image captured by the camera 1114 and received from the slave device 1100.

In FIG. 1, a structure in which the display and the controller are integrated into a single body in the master device 1200 is illustrated, but the present disclosure is not limited thereto, and the display and the controller may be configured as separate modules.

FIG. 2 illustrates a driving mechanism of a slave device according to one embodiment.

A driving structure of the slave device 1100 may include a guide tube holder 1105, a wire 1106, a medical tool 1107 (e.g., a basket or laser fiber), an endoscope 1110, and an access sheath 1120. The endoscope 1110 may include a main body 1111, a handle 1112, a guide tube 1113, and a camera 1114. The access sheath 1120 may include a sheath holder 1121 and a sheath 1122.

The slave device 1100 may be configured in a form in which the driving body 1102 equipped with the rail 1103, the driving unit 1104, the guide tube holder 1105, the wire 1106, the medical tool 1107, the camera 1114, the endoscope 1110, and the access sheath 1120 is disposed on an upper part of the support body 1101 that is movable relative to the ground. The driving body 1102 may move relatively to the support body 1101 for the procedure. For example, the driving body 1102 may receive a signal from the master device 1200 and translate in the horizontal and vertical directions relative to the support body 1101. The driving body 1102 may yaw relative to the support body 1101.

The rail 1103 may be formed on the driving body 1102 so that the driving unit 1104 that drives the endoscope may slide in the longitudinal direction of the driving body 1102. The driving unit 1104 may support the endoscope 1110 and control the endoscope 1110 by receiving a signal from the master device 1200. For example, the driving unit 1104 may move the handle 1112 of the endoscope 1110 that controls a direction in which the guide tube 1113 of the endoscope 1110 bends. When the endoscope 1110 is supported by the driving unit 1104, relative movement of the endoscope 1110 to the driving unit 1104 may be limited. Due thereto, the slave device 1100 may improve the stability and accuracy of the procedure by reducing errors resulting from movement of the endoscope 1110. Additionally, the driving unit 1104 may include an encoder (or a motor including an encoder), and the processor of the master device 1200 may utilize information about the encoder of the driving unit 1104.

The endoscope 1110 may include the main body 1111, the handle 1112, and the guide tube 1113. The endoscope 1110 may control bending of the guide tube 1113 from relative movement of the handle 1112 to the main body 1111 of the endoscope 1110. Accordingly, the guide tube 1113 may be inserted into the ureter for calculus removal. This may mean that the distal end portion of the guide tube 1113 bends and moves toward the location of a calculus.

The guide tube holder 1105 may support the guide tube 1113 of the endoscope 1110. The guide tube holder 1105 may prevent the guide tube 1113 of the endoscope 1110 from buckling when the driving unit 1104 slides or rotates relative to the driving body 1102. A plurality or guide tube holders 1105 may be provided, for example, along the longitudinal direction of the driving body 1102, and may be spaced apart from each other.

The access sheath 1120 may include the sheath holder 1121 and the sheath 1122.

The sheath holder 1121 may mount the sheath 1122 to an end portion of the driving body 1102. The sheath 1122 may be mounted inside a ureter U and guide the guide tube 1113. Accordingly, the guide tube 1113 may be movable relative to the sheath 1122 and reach a surgical site of the patient P by passing through the inside of the sheath 1122.

FIG. 3 illustrates a guide tube, a camera, and a medical tool inserted into a ureter according to one embodiment.

Additionally, the guide tube 1113 may guide the wire 1106 and the medical tool 1107 (e.g., the basket) disposed therein to a site where a calculus S is located.

The wire 1106 may be movable along the longitudinal axis (or longitudinal direction) of the guide tube 1113 within the guide tube 1113. The wire 1106 may be formed by covering a core with a structure in which a spring steel wire is spirally wound around a steel wire or with a flexible tube having high elasticity and flexibility, such as a Teflon tube, and joining another structure to a distal end portion of the steel wire according to a required work (e.g., joining the basket and a semicircular steel wire with high elasticity and flexibility). The wire 1106 may be coated with Teflon on the entire outside thereof to be movable smoothly, thereby preventing the wire from breaking or bending excessively within the blood vessel during the procedure and thus preventing damage to the blood vessel. The wire 1106 may be moved by the driving unit 1104 that moves the endoscope 1110 or may be moved through another driving unit configuration.

The medical tool 1107 may be detachable and may be configured in a form where only one of the basket and the laser fiber so that they are mounted alternately. However, the present disclosure is not limited thereto, and the medical tool may also be configured in a form where both the basket and the laser fiber are mounted simultaneously. The basket, which is an example of the medical tool 1107, may be connected to a distal end portion of the wire 1106 to be exposed out of the guide tube 1113, and may grip the calculus S by switching between an expanded state and a contracted state. The basket may be made of a material that returns to its original shape when no external force is applied, for example a shape memory alloy.

The medical tool 1107, for example, the basket and the laser fiber, may be mounted on a tool driving unit to be movable forward and backward.

The camera 1114 is a part of the configuration of the endoscope 1110 and may capture a medical image at the distal end portion of the guide tube 1113. Accordingly, the camera 1114 may capture a state in which the medical tool 1107 approaches a calculus inside an organ (e.g., a kidney) to either grip the calculus or not, or to either disintegrate the calculus or not. The camera 1114 may be fixed to the distal end portion of the guide tube 1113. Since the medical tool 1107 is movable (e.g., movable forward or backward) relative to the guide tube 1113, a distance between the medical tool 1107 and the camera 1114 may vary depending on manipulation of the user 1900. In this specification, a distance that serves as a criterion for determining the size of the guidance graphic representation is a distance from the camera 1114 to the medical tool 1107, i.e., a distance between the camera 1114 and the medical tool 1107.

FIG. 4 exemplarily illustrates a basket gripping a calculus through the system for providing guidance for calculus removal according to one embodiment.

When the medical tool 1107 is, for example, the basket, the processor may estimate a distance d from the distal end portion of the guide tube 1113 (e.g., the position where the camera 1114 is capturing) to the medical tool 1107 (e.g., the basket) gripping a calculus S to determine the size of the guidance graphic representation. The distance d may also refer to a length that the wire 1106 is exposed from the distal end portion of the guide tube 1113. The above may equally be applied to a case where the medical tool is the laser fiber. Accordingly, the processor may infer an image-based distance between the medical tool and the distal end portion of the guide tube 1113 from the captured medical image, and estimate an encoder-based distance between the medical tool and the distal end portion of the guide tube 1113 from the information about the encoder of the driving unit 1104. The encoder-based distance may be estimated, for example, as a value (e.g., having a linear form) corresponding to a predetermined number of encoder rotations. The processor may then determine an estimated distance on the basis of the image-based distance and the encoder-based distance.

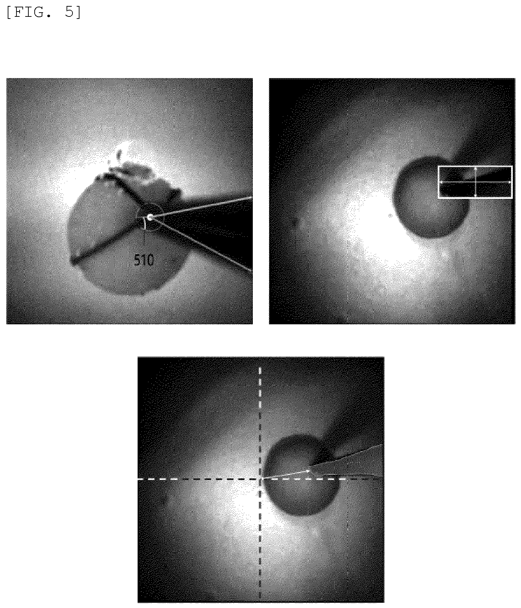

FIG. 5 exemplarily illustrates a basket gripping a calculus to infer an image-based distance in the system for providing guidance for calculus removal according to one embodiment. Also, a laser fiber approached a calculus to infer an image-based distance in the system for providing guidance for calculus removal according to one embodiment is exemplarily illustrated. Specifically, the image-based distance may be inferred from an X or Y or diagonal length of a laser fiber bounding box region, or a length from a boundary of a medical image to a distal end portion of the laser fiber. Additionally, as a specific example, the image-based distance may be inferred from a pixel distance between the distal end portion of the laser fiber and a center of a screen on the medical image and an area of a laser fiber region.

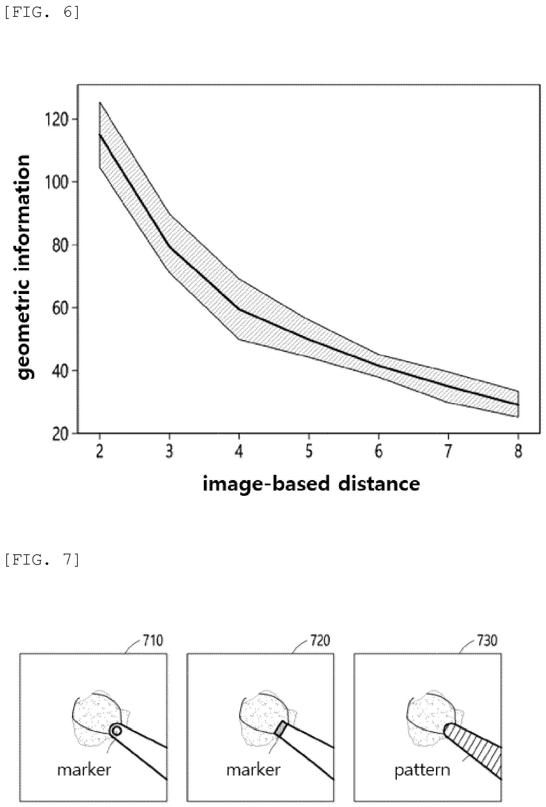

FIG. 6 exemplarily illustrates a graph of an image-based distance corresponding to geometric information used in the system for providing guidance for calculus removal according to one embodiment.

When the medical tool is the basket, the processor may estimate geometric information of a shaft from a shaft region of the basket from the captured medical image. The geometric information may be estimated on the basis of an average detected length of a bounding box. Additionally, the processor may infer the image-based distance from the estimated geometric information on the basis of a machine learning model, for example, an object detection or instance segmentation algorithm. The machine learning model is a model designed and trained to estimate the geometric information from the medical image, and may include, for example, a neural network. Without being limited thereto, the processor may infer the image-based distance by inputting the medical image into another machine learning model or a fitted function. For example, the image-based distance may be inferred via a fitted function (e.g., a polynomial regression function) or a trained model on the basis of geometric information corresponding to each collected image-based distance. The other machine learning model is a model designed and trained to directly estimate the image-based distance from the medical image, and may include, for example, a neural network.

Additionally, the geometric information is a component value that does not change or is insensitive to changes or robust to factors other than a distance between the medical tool and a distal end portion of the wire on the medical image. The geometric information of the shaft may include at least one of an area occupied by the shaft region of the basket on the medical image, a diameter of an estimated circle corresponding to a distal end portion of the shaft, a radius of the estimated circle corresponding to the distal end portion of the shaft, a length from a boundary of the medical image to the distal end portion of the shaft, a length from a center of the medical image to the distal end portion of the shaft, and an inclination of the shaft. For example, the shaft region of the basket may be a region corresponding to a part of the wire connected to the basket on the medical image. The geometric information about the shaft may include, for example, a diameter of an estimated circle corresponding to the distal end portion of the shaft or a radius 510 of the estimated circle corresponding to the distal end portion of the shaft, but the present disclosure is not limited thereto.

FIG. 7 exemplarily illustrates markers provided on a medical tool used in the system for providing guidance for calculus removal according to one embodiment.

Additionally, a marker or pattern may be attached or mounted on the medical tool (e.g., the basket) to facilitate extraction of the geometric information from the shaft region of the basket. A marker 710 may be formed in a circular shape on a part of the shaft region, a marker 720 may be formed in a strip shape surrounding the shaft region, or a marker 730 may be formed in a patterned shape on the shaft region. Due thereto, the processor may quickly recognize the more prominent shaft region of the basket and thus extract the geometric information for the image-based distance more quickly.

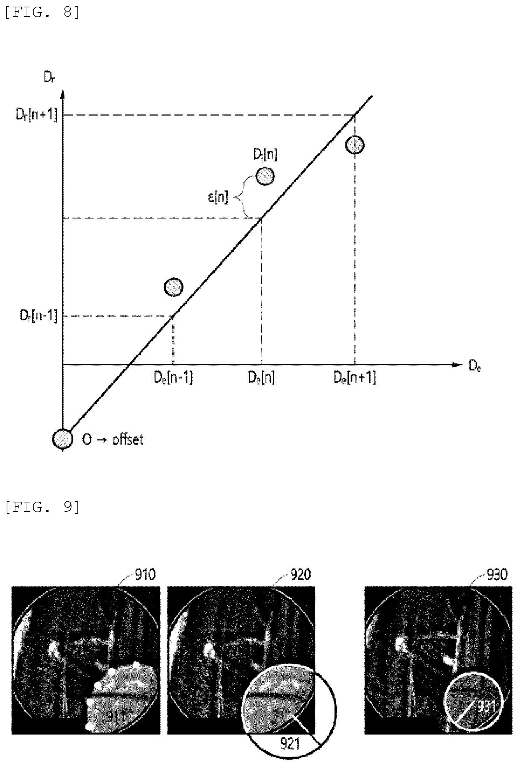

FIG. 8 exemplarily illustrates a graph of relationship between an image-based distance, an encoder-based distance, and an estimated distance in the system for providing guidance for calculus removal according to one embodiment.

The encoder-based distance may have errors in the low frequency band, and the image-based distance may have errors in the high frequency band. Therefore, the encoder-based distance value and the image-based distance value may each have a different error component, so they may be used complementary to each other. The processor may determine the estimated distance by applying a bias compensation value determined on the basis of the image-based distance and the encoder-based distance to the encoder-based distance. The processor may determine the estimated distance using Equation 1.

[Equation 1]

$$D_r[n] = D_e[n] - \frac{\sum_{m=1}^{n}\left[D_e[m] - D_i[m]\right]}{n}$$

Here, $D_r$ represents the estimated distance, $D_e$ represents the encoder-based distance, and $D_i$ represents the

$$\frac{\sum_{m=1}^{n}\left[D_e[m] - D_i[m]\right]}{n}$$

image-based distance. may represent a value obtained by accumulating expected values based on the difference between the encoder-based distance and the image-based distance, calculating a sum of the accumulated expected values, and dividing the sum by the number of accumulations. This may represent an offset value, i.e. an error value (e.g., a bias compensation value). The bias compensation value may have a value that converges over time (e.g., to a certain constant value) after calculation of the estimated distance is started.

FIG. 9 exemplarily illustrates a function fitting method for deriving a graphic representation radius used in the system for providing guidance for calculus removal according to one embodiment.

FIG. 10 exemplarily illustrates a graph of a graphic representation radius corresponding to an estimated distance used in the system for providing guidance for calculus removal according to one embodiment.

The estimated distance may be input into an nth order polynomial function to determine a size (e.g., a radius) of a corresponding guidance graphic representation. The size of the guidance graphic representation may be a criterion for safely removing a calculus at the corresponding estimated distance. Therefore, the calculus may be safely removed when it is within the size of the guidance graphic representation. This may also mean that the size of the guidance graphic representation may be defined on the basis of the size of the sheath 1122 that guides the guide tube 1113. Fitting an nth-order polynomial function may be achieved as follows. First, an image according to a tool distance (e.g., in mm) for a gripped calculus.

Here, the process illustrated in FIG. 9 may be performed in a phantom model which simulates the human kidney and urethra, and a calculus placed in the phantom model may have a physically smaller size than the sheath. In other words, the calculus captured on medical images in FIG. 9 may have a size that can be protected by the sheath while being gripped by the basket.

For example, in a method 910, an outer edge of a calculus in each image may be marked with points 911, and in methods 920 and 930, a radius value for each tool distance obtained by fitting it to a circle may be fitted to an nth-order polynomial function. Accordingly, when a radius 921 has a greater value than a radius 931, the estimated distance may be set smaller than the radius 931. This also implies that the size of the guidance may also change as the estimated distance increases or decreases as the basket moves.

FIG. 11 exemplarily illustrates a change in a guidance graphic representation that is expressed differently according to a changing estimated distance in the system for providing guidance for calculus removal according to one embodiment.

The processor may reduce the size of the guidance graphic representation (112) as the estimated distance increases (D1) from a current basket location (111). Therefore, for example, even when the basket approaches (e.g., the estimated distance increases) a calculus to grip it, rather than necessarily gripping the calculus, whether the calculus is of an appropriate size may be determined by the guidance displayed. This may also be more useful for the laser fiber. This is because a size that needs to be disintegrated may be determined in advance before contact with the calculus, enabling a quick procedure.

The processor may increase the size of the guidance graphic representation (113) when the estimated distance decreases (D2) from the current medical tool location (111). Therefore, for example, even when the basket moves to grip and remove the calculus (e.g., the estimated distance decreases) or the laser fiber moves to disintegrate the calculus, the guidance graphic representation may clearly indicate a particular size that is extractable or needs to be disintegrated, making it possible to more accurately determine whether removal is possible or disintegration is necessary from a distant location.

When the medical tool 1107 is the laser fiber, the processor may sense a moment when the distal end portion of the laser fiber makes contact with the calculus, but the present disclosure is not limited thereto. For example, when the user 1900 determines that the laser fiber is making contact with the calculus, the user 1900 may manually input information regarding contact into the master device 1200. When sensing contact of the laser fiber, the processor may capture and store a target image at a time point where the distal end portion of the laser fiber makes contact with the calculus. The processor may output the medical image to the display of the master device 1200 simultaneously with the target image. Due thereto, the laser fiber may determine whether disintegration is necessary on the basis of the guidance graphic representation on the medical image, thereby performing an appropriate amount of disintegration and enabling rapid calculus removal using the basket.

Additionally, the processor may receive an input from the user 1900 to select a point on the target image and, in response thereto, output the guidance graphic representation on the target image on the basis of the selected point. This may mean that, for example, when the guidance graphic representation is output at a misaligned location on the target image, the user 1900 moves the location of the guidance graphic representation by selecting a desired point using a touch input. In another method, the processor may detect a calculus region on the target image and output the guidance graphic representation on the target image on the basis of a reference point in the detected calculus region. Due thereto, the user 1900 may easily determine the size of the calculus without the hassle of having to make a touch input.

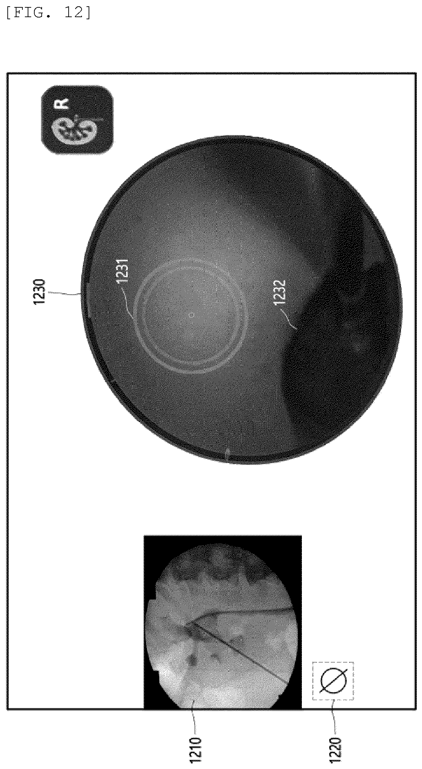

FIG. 12 exemplarily illustrates a screen of a display on which a guidance graphic representation is output in the system for providing guidance for calculus removal according to one embodiment.

A region 1210 is a region that displays procedure information, and may be, for example, a region that outputs an X-ray image obtained by capturing a site where a calculus is present in advance or an image that displays the distance and location of the medical tool 1107 entering the body in real time. A region 1220 is a region that controls on/off of the guidance graphic representation, and in addition to turning the guidance graphic representation on/off when necessary, it may also manually start operation of the system for providing guidance to provide the guidance graphic representation. This eliminates the need for providing the guidance graphic representation at locations other than the region where the calculus is present, thereby enabling efficient use of the processor within the master device 1200. A region 1230 is a region where the medical image captured by the camera 1114 is output, and may display the guidance graphic representation (1231) and the medical tool 1107 (1232).

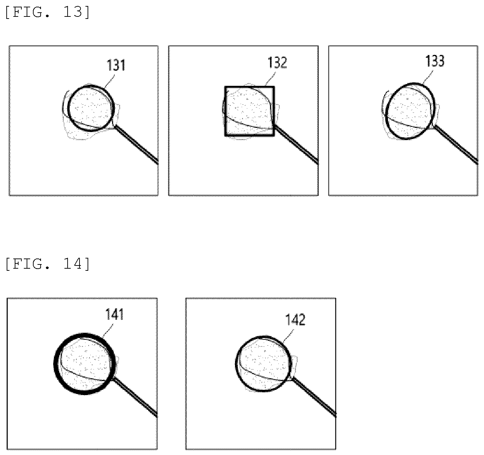

FIG. 13 exemplarily illustrates types of guidance graphic representations in the system for providing guidance for calculus removal according to one embodiment.

The guidance graphic representation may be expressed as one of circular, oval, square, polygonal, and closed curve shapes, but the present disclosure is not limited thereto. For example, in the case of a calculus of general shape, a circular guidance graphic representation 131 may be sufficient, but in the case of an abnormal shape, such as a calculus that is elongated in one direction, a square guidance graphic representation 132 or an oval guidance graphic representation 133 may be more appropriate.

FIG. 14 exemplarily illustrates a thickness of a boundary of a guidance graphic representation in the system for providing guidance for calculus removal according to one embodiment.

The boundary of the guidance graphic representation may have a predetermined thickness. Accordingly, the processor may reduce the thickness of the boundary of the guidance graphic representation when reliability of the estimated distance increases over time. This may indicate that a guidance graphic representation with a boundary thickness 142 that is smaller than a boundary thickness 141 of an initially displayed guidance graphic representation is a more accurate guidance graphic representation. This representation may also be performed vice versa (e.g., the boundary of the guidance graphic representation may become thicker over time). Additionally, the reliability of the estimated distance may be determined on the basis of the bias compensation value in Equation 1 above.

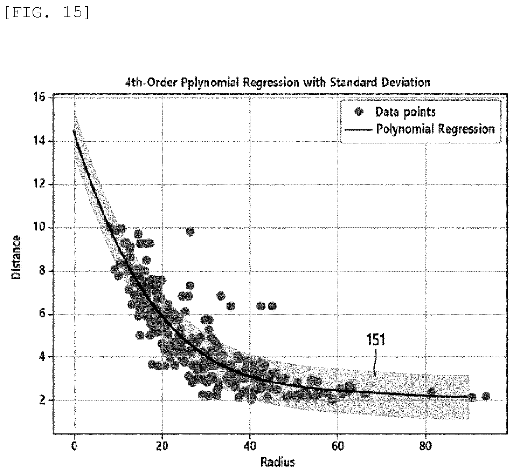

FIG. 15 exemplarily illustrates a graph of results of verifying a polynomial regression function used to infer an image-based distance in the system for providing guidance for calculus removal according to one embodiment.

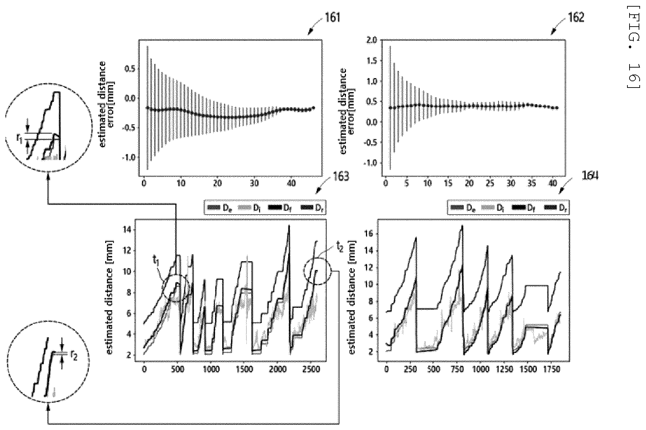

FIG. 16 exemplarily illustrates graphs of an encoder-based distance value obtained by applying a bias compensation value used to calculate an estimated distance according to the size and time of a calculus in the system for providing guidance for calculus removal according to one embodiment, and comparison results between a distance estimated using each image estimation method and a real distance.

Image-based distances inferred using the polynomial regression function are mostly distributed within a normal range 151, so it can be seen that accurate image-based distances are inferred except for errors in the low frequency band.

Additionally, estimated distance determination based on the image-based distance and the encoder-based distance becomes more accurate as the variance (e.g., error) of the bias compensation value becomes smaller. Since the variance of the bias compensation value becomes smaller as the number of accumulated values increases over time, the estimated distance determined may become more accurate over time. A graph 161 represents a cumulative average value of image-based distance errors for a small calculus, and a graph 162 represents a cumulative average value of image-based distance errors for a large calculus. In the case of the large calculus, the cumulative average value tends to quickly converge to 0, but after a certain period of time, the cumulative average value converges to 0 even in the case of the small calculus. This may mean that the estimated distance determined is time-dependent rather than calculus size-dependent.

Additionally, a graph 163 represents an estimated distance determined for each method for the small calculus, and a graph 164 represents an estimated distance determined for each method for the large calculus. An error of an encoder-based distance $D_e$ to a real distance $D_r$ may be generally determined to be the highest value, and an error of an image-based distance $D_i$ to the real distance $D_r$ may be determined to be a lower value. On the other hand, an estimated distance $D_r$ determined using the system for providing guidance according to the embodiment may be determined as a result value most similar to the real distance $D_r$ compared to the encoder-based distance De and the image-based distance

$D_i$. This may mean that the user 1900 more accurately determines whether a calculus is removable through the guidance graphic representation generated on the basis of an accurate distance. Additionally, in terms of the accuracy of the estimated distance $D_f$ over time, a difference $r_1$ between the estimated distance $D_f$ and the real distance $D_r$ at a later time $t_2$ is smaller than a difference $r_1$ between the estimated distance $D_f$ and the real distance $D_r$ at an early time $t_1$, so a more accurate estimated distance may be determined over time.

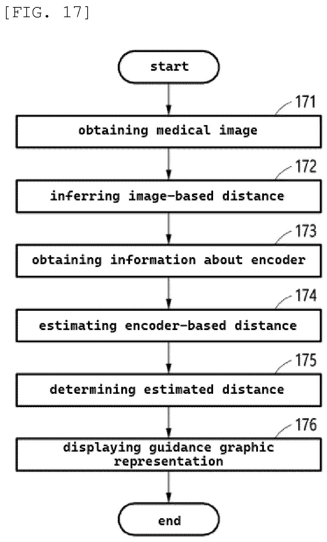

FIG. 17 illustrates a flowchart of a method of providing guidance for calculus removal according to one embodiment.

In step 171, a processor may obtain a medical image captured by including a medical tool at a distal end portion of a guide tube inserted into the ureter. The medical image may be obtained automatically, for example, during operation of the medical tool (e.g., gripping of a basket), obtained when a user inputs an on-command to output a guidance graphic representation, or obtained automatically according to a predetermined extension length of the guide tube, but the present disclosure is not limited thereto.

At step 172, the processor may infer an image-based distance between the medical tool and a camera at the distal end portion of the guide tube from the captured medical image. For example, when the medical tool is a basket, the processor may estimate geometric information about a shaft from a shaft region of the basket from the captured medical image. After that, the processor may infer the image-based distance from the estimated geometric information on the basis of a machine learning model.

In step 173, the processor may obtain information about an encoder of a driving unit that moves a wire within the guide tube.

At step 174, the processor may estimate an encoder-based distance between the medical tool and the camera at the distal end portion of the guide tube from the information about the encoder.

At step 175, the processor may determine an estimated distance between the medical tool and the camera at the distal end portion of the guide tube on the basis of the image-based distance and the encoder-based distance. The processor may determine the estimated distance by applying a bias compensation value determined on the basis of the image-based distance and the encoder-based distance to the encoder-based distance. Here, the bias compensation value may have a value that converges over time after calculation of the estimated distance is started.

In step 176, the processor may display a guidance graphic representation of a size determined on the basis of the estimated distance by overlaying it on the medical image. The processor may adjust the size of the guidance graphic representation on the basis of a changing estimated distance between the medical tool and the camera at the distal end portion of the guide tube. The size of the guidance graphic representation may decrease as the estimated distance increases and may increase as the estimated distance decreases.

After step 176, the processor may sense a user's external input and change the position of the guidance on the medical image in response to the user's external input.

Additional Examples

Example 1. A guidance provision device inserted into a target, the guidance provision device including the following:

  * a guide tube that is inserted into the target,
  * a wire that is movable within the guide tube,
  * a driving unit that moves the wire, and
  * a processor that determines a size of an object within the target.

Example 2. The guidance provision device of any of the Examples above, wherein the wire is movable along the longitudinal axis of the guide tube.

Example 3. The guidance provision device of any of the Examples above, wherein the driving unit is coupled to a distal end portion of the wire.

Example 4. The guidance provision device of any of the Examples above, further including: a medical tool exposed out of the guide tube and a camera capturing an image at a distal end portion of the guide tube.

Example 5. The guidance provision device of any of the Examples above, wherein the processor determines the size of the object on the basis of an estimated distance between the medical tool and the distal end portion of the guide tube.

Example 6. The guidance provision device of any of the Examples above, wherein the guide tube has a predetermined size.

Example 7. The guidance provision device of any of the Examples above, further including: a master device that displays a graphic representation by overlaying it on a medical image captured by the camera.

Example 8. The guidance provision device of any of the Examples above, wherein the processor estimates an estimated distance between the medical tool and the distal end portion of the guide tube from information about an encoder of the driving unit, and determines the estimated distance on the basis of an image-based distance and the encoder-based distance.

Example 9. The guidance provision device of any of the Examples above, wherein the medical tool is a basket including a shaft region and a shaft, and the processor estimates geometric information about the shaft of the basket from the captured medical image and infers the image-based distance from the estimated geometric information on the basis of a machine learning model.

Example 10. The guidance provision device of any of the Examples above, wherein the geometric information about the shaft includes at least one of an area occupied by the shaft region of the basket on the medical image, a diameter of an estimated circle corresponding to a distal end portion of the shaft, a radius of the estimated circle corresponding to the distal end portion of the shaft, a length from a boundary of the medical image to the distal end portion of the shaft, a length from a center of the medical image to the distal end portion of the shaft, and an inclination of the shaft.

Example 11. The guidance provision device of any of the Examples above, wherein the processor infers the image-based distance by inputting the medical image into another machine learning model.

Example 12. The guidance provision device of any of the Examples above, wherein the processor applies a bias compensation value to calculate the estimated distance on the basis of the image-based distance and the encoder-based distance, wherein the bias compensation value is determined on the basis of the encoder-based distance.

Example 13. The guidance provision device of any of the Examples above, wherein the bias compensation value has a value that converges over time after calculation of the estimated distance is started.

Example 14. The guidance provision device of any of the Examples above, wherein the processor reduces a size of the guidance graphic representation as the estimated distance increases, and increases the size of the guidance graphic representation as the estimated distance decreases.

Example 15. The guidance provision device of any of the Examples above, wherein the medical tool is a laser fiber.

Example 16. The guidance provision device of any of the Examples above, wherein when the medical tool is the laser fiber, the processor senses a moment when the laser fiber makes contact with the object.

Example 17. The guidance provision device of any of the Examples above, wherein when the medical tool is the laser fiber, the processor captures and stores a target image at a time point where a distal end portion of the laser fiber makes contact with the object.

Example 18. The guidance provision device of any of the Examples above, wherein when the medical tool is the laser fiber, the processor outputs the medical image to a display of the master device simultaneously with the target image.

Example 19. The guidance provision device of any of the Examples above, wherein the processor receives an input from a user to select a point on the target image, and outputs the guidance graphic representation on the target image on the basis of the selected point.

Example 20. The guidance provision device of any of the Examples above, wherein the processor detects a calculus region on the target image and outputs the guidance graphic representation on the target image on the basis of a reference point in the detected calculus region.

Example 21. The guidance provision device of any of the Examples above, wherein the guidance graphic representation has a shape of circular, oval, square, polygonal, or closed curve.

Example 22. The guidance provision device of any of the Examples above, wherein the size of the guidance graphic representation is defined on the basis of a capping size of the basket of the medical tool.

Example 23. The guidance provision device of any of the Examples above, wherein a boundary of the guidance graphic representation has a large thickness, and as reliability of the estimated distance increases over time, the thickness of the boundary of the guidance graphic representation decreases.

Example 24. The guidance provision device of any of the Examples above, wherein the processor performs the following operations:

* obtaining the medical image captured from the distal end portion of the guide tube having the medical tool while the guide tube is inserted into the ureter,
* inferring the image-based distance between the medical tool and the distal end portion of the guide tube from the captured medical image,
* obtaining the information about the encoder of the driving unit for moving the wire within the guide tube,
* estimating the encoder-based distance between the medical tool and the distal end portion of the guide tube from the information about the encoder,
* determining the estimated distance between the medical tool and the distal end portion of the guide tube on the basis of the image-based distance and the encoder-based distance, and
* displaying the guidance graphic representation of a size determined on the basis of the estimated distance by overlaying it on the medical image.

Example 25. The guidance provision device of any of the Examples above, wherein the guidance provision device has a function of sensing a user's input after the guidance graphic representation is displayed overlaid on the medical image.

Example 26. The guidance provision device of any of the Examples above, wherein the guidance provision device changes the position of the guidance on the medical image in response to the user's input.

Example 27. The guidance provision device of any of the Examples above, wherein the guidance provision device is used during surgery.

Example 28. A guidance provision system, including the following:

* a master device that provides a signal, and
* a guidance provision device that is inserted into a target, wherein the guidance provision device determines a size of an object within the target in response to a signal from the master device.

Example 29. The guidance provision system of Example 28, wherein the guidance provision device includes the following:

* a guide tube that is inserted into the target,
* a wire that is movable within the guide tube,
* a driving unit that moves the wire, and
* a processor that determines a size of an object.

Example 30. The guidance provision system of Example 28 or 29, wherein the wire is movable within the guide tube along the longitudinal axis of the guide tube.

Example 31. The guidance provision system of any of Example 28 to 30, wherein the driving unit is coupled to a distal end portion of the wire.

Example 32. The guidance provision device of any of the Examples above, further including: a medical tool exposed out of the guide tube and a camera capturing an image at a distal end portion of the guide tube.

Example 33. The guidance provision system of any of Examples 28 to 32, wherein the processor determines the size of the object on the basis of an estimated distance between the medical tool and the distal end portion of the guide tube.

Example 34. The guidance provision system of any of Examples 28 to 33, wherein the guide tube has a predetermined size.

Example 35. The guidance provision system of any of Examples 28 to 34, further including: a master device that displays a graphic representation by overlaying it on a medical image captured by the camera.

Example 36. The guidance provision system of any of Examples 28 to 35, wherein the processor estimates an encoder-based distance between the medical tool and the distal end portion of the guide tube from information about an encoder of the driving unit for moving the wire, and determines the estimated distance on the basis of an image-based distance and the encoder-based distance.

Example 37. The guidance provision system of any of Examples 28 to 36, wherein the medical tool is a basket, and the processor estimates geometric information about a shaft of the basket from the captured medical image and infers the image-based distance from the estimated geometric information on the basis of a machine learning model.

Example 38. The guidance provision system of any of Examples 28 to 37, wherein the geometric information about the shaft of the basket includes one or more of the following: an area of occupied by a shaft region of the basket, a diameter of an estimated circle corresponding to a distal end portion of the shaft, a radius of the estimated circle corresponding to the distal end portion of the shaft, a length from the distal end portion to a boundary of the medical image, a length from a center of the medical image to the distal end portion of the shaft, and an inclination of the shaft.

Example 39. The guidance provision system of any of Examples 28 to 38, wherein the processor infers the image-based distance by inputting the medical image into another machine learning model.

Example 40. The guidance provision system of any of Examples 28 to 39, wherein the processor determines the estimated distance by applying a bias compensation value, wherein the bias compensation value is determined on the basis of the image-based distance and the encoder-based distance for the encoder-based distance.

Example 41. The guidance provision system of any of Examples 28 to 40, wherein the bias compensation value has a value that converges over time after calculation of the estimated distance is started.

Example 42. The guidance provision system of any of Examples 28 to 41, wherein the processor reduces a size of the guidance graphic representation as the estimated distance increases, and increases the size of the guidance graphic representation as the estimated distance decreases.

Example 43. The guidance provision system of any of Examples 28 to 42, wherein the medical tool is a laser fiber.

Example 44. The guidance provision system of any of Examples 28 to 43, wherein when the medical tool is the laser fiber, the processor senses a moment when the laser fiber makes contact with the object.

Example 45. The guidance provision system of any of Examples 28 to 44, wherein when the medical tool is the laser fiber, the processor captures and stores a target image at a time point where a distal end portion of the laser fiber makes contact with the object.

Example 46. The guidance provision system of any of Examples 28 to 45, wherein when the medical tool is the laser fiber, the processor outputs the medical image to a display of the master device simultaneously with the target image.

Example 47. The guidance provision system of any of Examples 28 to 46, wherein the processor receives an input from a user to select a point on the target image, and outputs the guidance graphic representation on the target image on the basis of the selected point.

Example 48. The guidance provision system of any of Examples 28 to 47, wherein the processor detects a calculus region on the target image and outputs the guidance graphic representation on the target image on the basis of a reference point in the detected calculus region.

Example 49. The guidance provision system of any of Examples 28 to 48, wherein the guidance graphic representation has a shape of circular, oval, square, polygonal, or closed curve.

Example 50. The guidance provision system of any of Examples 28 to 49, wherein the size of the guidance graphic representation is defined on the basis of a capping size of the basket of the medical tool.

Example 51. The guidance provision system of any of Examples 28 to 50, wherein a boundary of the guidance graphic representation has a large thickness, and as reliability of the estimated distance increases over time, the thickness of the boundary of the guidance graphic representation decreases.

Example 52. The guidance provision system of any of Examples 28 to 51, wherein the processor performs the following operations:

* obtaining the medical image captured from the distal end portion of the guide tube having the medical tool while the guide tube is inserted into the ureter,
* inferring the image-based distance between the medical tool and the distal end portion of the guide tube from the captured medical image,
* obtaining the information about the encoder of the driving unit for moving the wire within the guide tube,
* estimating the encoder-based distance between the medical tool and the distal end portion of the guide tube from the information about the encoder,
* determining the estimated distance between the medical tool and the distal end portion of the guide tube on the basis of the image-based distance and the encoder-based distance, and
* displaying the guidance graphic representation of a size determined on the basis of the estimated distance by overlaying it on the medical image.

Example 53. The guidance provision system of any of Examples 28 to 52, wherein the master device includes an input device for manually controlling the guidance representation overlaid on the image.

Example 54. The guidance provision system of any of Examples 28 to 53, wherein the user adjusts the position of the guidance representation on the image using the input device.

Example 55. The guidance provision system of any of Examples 28 to 54, wherein the guidance provision system is used to provide guidance during surgery.

The above examples describe implementations of a guidance provision device and system in the medical field, which helps improve the accuracy and efficiency of medical procedures. The device and system may be used to support target manipulation, increase safety, and assist the work of medical professionals.

Example 56: A surgical navigation system, wherein a guidance device includes a tactile feedback function based on the position and movement of a surgical tool. This enables a user to perform surgery while receiving tactile sensations.

Example 57: The surgical navigation system, wherein a tracking sensor is attached to an endoscope to monitor its position and orientation within the body. The system provides real-time visual feedback to help a surgeon navigate the endoscope and precisely manipulate it to target a specific site.

Example 58: A robotic surgical system, including a guidance module that uses pre-surgical image data to create a virtual anatomical model of a patient. This enables a surgeon to plan precisely and manipulate a surgical tool in a virtual environment before surgery.

Example 59: An autonomous surgical robotic system analyzes the vital signs of a patient and the movement of a surgical tool in real time using an AI algorithm and a sensor. This enables a robot to perform safe and accurate surgery, and enables a surgeon to receive automated support from the system.

Example 60: An innovative medical image guidance system processes an image such as a CT scan image or a magnetic resonance imaging (MRI) image in real time to extract anatomical information. Based on this information, the system provides precise positioning guidance to a surgeon and provides a real-time updated image during surgery.

Example 61: An ultrasound guidance system generates a real-time image of an internal organ using an ultrasound sensor and image processing technology. This enables a surgeon to determine and manipulate the exact location during surgery and perform safe and precise surgery.

Example 62: A surgical guidance system using virtual reality (VR), wherein the system helps a surgeon simulate and practice surgery by virtually reproducing a patient's anatomical model. This enables the surgeon to make a precise plan prior to actual surgery, improving the safety and efficiency of surgery.

The above various methods may be used to provide guidance during surgery and to assist a surgeon in his or her work. Each system has its own unique features and advantages and may be developed and applied to suit a variety of situations and needs in the medical field.

Example 63: A real-time surgical guidance system that helps a surgeon view and manipulate an image in real time during surgery using a camera and image processing technology. This enables the surgeon to more accurately visualize a surgical procedure and quickly perform any necessary manipulation.

Example 64: A robot-assisted surgical system assists a surgeon in his or her manipulation using a robot arm and a tool. The robot arm performs precise and stable movements and transmits surgeon's manipulation more accurately. This increases the precision and safety of surgery.

Example 65: A microscope system supports observation and manipulation of a surgical procedure using a small, precise microscope. This enables a surgeon to perform precise and safe work even in surgeries requiring micromanipulation.

Example 66: A surgical guidance system using a neural network, wherein the system predicts and guides the movement of a surgical tool using a neural network algorithm. This enables a surgeon to anticipate the next movement of the surgical tool and optimize manipulation.

Example 67: A real-time position tracking system tracks and guides the position of a surgical tool in real time using a sensor and position tracking technology. This enables a surgeon to obtain accurate positional information during surgery and perform safe and precise manipulation.

Example 68: An automated surgical guidance system automates and guides a surgical procedure by combining an AI algorithm and robotics technology. This enables a surgeon to achieve accurate and consistent results through automated manipulation of a surgical tool, reducing surgical time and costs.

Example 69: A surgical robotic system assists a surgeon in performing surgery remotely using a robot arm and a sensor. This enables to overcome geographical limitations and provide professional medical services.

Example 70: With the advancement of modern medical technology, a surgical guidance system continues to advance and evolve. A more accurate and faster guidance system is expected to be developed in the future.

Example 71: Application of a surgical guidance system not only improves the precision and safety of surgery, but also increases the work efficiency of medical professionals. This has a positive impact on improving treatment effectiveness and comfort for patients.

Example 72: A surgical guidance system promotes collaboration and knowledge sharing in the medical field. This system enables medical professionals to share the latest technologies and information, and collaborate to develop and apply more effective treatment methods.

Example 73: A surgical guidance system contributes to providing patient-centered medical services along with innovations in medical technology. This enables patients to undergo more accurate and safer surgery and minimize recovery time.

Example 74: A surgical guidance system is recognized as one of the representative future technologies in the medical field. Through continuous research and development, a more advanced system is expected to be developed, bringing greater innovation and progress to the medical field.

Example 75: Application of an innovative surgical guidance system means pushing the boundaries of medical technology. This provides better medical services to medical professionals and patients, and serves as a major driving force for medical innovation.

Example 76: A surgical guidance system offers exciting prospects for the future of medical technology. A more advanced guidance system is expected to be developed in the medical field through convergence with technologies such as real-time image processing, artificial intelligence, and robotics.

Example 77: Various surgical guidance systems and methods introduced so far are one of the major interests in research and development in the medical field. A more diverse and innovative surgical guidance system is expected to emerge in the future along with the advancement of medical technology.

Example 78: Application of a surgical guidance system plays a vital role in the medical field, where patient safety and health are given top priority. With the advancement of technology and the efforts of medical professionals, the surgical guidance system is expected to continuously advance and evolve.

Example 79: A surgical guidance system is recognized as an essential tool that reduces medical costs and improves efficiency along with the advancement of medical technology. This helps patients receive more affordable and effective medical services.

Example 80: A surgical guidance system is one of the technologies representing digitalization and innovation in the medical field. With the advancement of medical technology, a more accurate and efficient surgical guidance system is expected to be developed and be a great help to the medical field.

Example 81: A surgical guidance system plays a major role in improving the quality of medical profession. This system enables medical professionals to perform precise surgery and protect the health and safety of patients.

Example 82: A surgical guidance system encourages and supports research and innovation in the medical field. This system, which contributes to the advancement of medical technology, provides innovative medical services to medical professionals and patients.

Example 83: Application of a surgical guidance system is attracting attention as part of the next-generation medical services in the medical field. Convergence of innovative technology and medical enables to provide more accurate and personalized medical services and improve patient treatment outcomes and satisfaction.

The above various surgical guidance systems and methods may contribute to supporting and improving surgical operations in the medical field more accurately and safely. A more advanced system is expected to be developed through advancements in medical technology and research.

The embodiments described above may be implemented using hardware components, software components, and/or a combination of hardware components and software components. For example, the device, method, and components described above may be implemented using, for example, a processor, a controller, an arithmetic logic unit (ALU), a digital signal processor, a micro-computer, a field programmable array (FPA), a programmable logic unit (PLU), a microprocessor, or a general-use or special purpose computer that may carry out or respond to instructions. A processing device may execute an operation system (OS) or a software application executable on the OS. Also, the processing device may access, store, manipulate, process, or generate data in response to the execution of the software application. For purpose of simplicity, a single processing device may be described. However, one of ordinary skill in the art may understand that the processing device may include multiple processing elements and/or multiple types of processing elements. For example, the processing device may include multiple processors or a processor and a controller. Additionally, the processing device may include a different processing configuration, such a parallel processor.

Software may include a computer program, a code, an instruction, or any combination thereof. The software may design a processing device to operate as desired and may independently or collectively send instructions to the processing device. Software and/or data may be stored by any type of machine, component, physical equipment, virtual equipment, or computer storage media or device in order to be read by a processing device or to provide instructions or data to the processing device. The software may be distributed over network-coupled computer systems so that the software is stored and executed in a distributed fashion. The software and data may be stored by computer-readable recording media.

The method described herein may be implemented as program instructions executable by various computer media and may be written to computer-readable recording media. The computer-readable recording media may individually or collectively include program instructions, data files, data structures, and the like. The program instructions included in the media may be specifically designed for the various embodiments or may be well known to one of ordinary skill in the art. Examples of the computer-readable recording media include magnetic storage media (e.g., floppy disks, hard disks, magnetic tapes, etc.), optical recording media (e.g., compact disc (CD)-ROMs, or digital versatile discs (DVDs)), magneto-optical media (e.g., floptical disks), and hardware devices (e.g., ROM, RAM, flash memory, etc.) configured to store and execute program instructions. Examples of the program instructions include machine language code produced by a compiler, and high-level language code executable by a computer using an interpreter.

The above-described hardware device may be configured to act as one or more software modules in order to perform the operations according to the various embodiments, and vice versa.

The embodiments described herein are to be considered in a descriptive sense only, and not for purposes of limitation. Descriptions of features or aspects in each embodiment are to be considered as being applicable to similar features or aspects in other embodiments. For example, suitable results may be achieved if the described techniques are performed in a different order, and/or if components in a described system, architecture, device, or circuit are combined in a different manner and/or replaced or supplemented by other components or their equivalents.

Therefore, it is to be appreciated that all variations within the scope of the claims and their equivalents are to be construed as being included in the disclosure.

**Claims**

1. A system for providing guidance for calculus removal, the system comprising:

    a slave device including a guide tube that is configured to be inserted into a ureter for calculus removal, a wire that is configured to be movable along a longitudinal axis of the guide tube within the guide tube, a driving unit that is configured to move the wire, a medical tool that is connected to a distal end portion of the wire to be exposed out of the guide tube, and a camera that is configured to capture a medical image at a distal end portion of the guide tube;

a processor configured to determine a size of a guidance graphic representation indicating a calculus size that is extractable or needs to be disintegrated by a basket or laser fiber on the basis of an estimated distance between the medical tool and the camera at the distal end portion of the guide tube; and

a master device configured to display the guidance graphic representation having the determined size by overlaying the guidance graphic representation on the medical image captured by the camera and received from the slave device.

2. The system of claim 1, wherein the processor infers an image-based distance between the medical tool and the camera at the distal end portion of the guide tube from the captured medical image,

the processor estimates an encoder-based distance between the medical tool and the camera at the distal end portion of the guide tube from information about an encoder of the driving unit, and

the processor determines the estimated distance on the basis of the image-based distance and the encoder-based distance.

3. The system of claim 2, wherein when the medical tool is the basket, the processor estimates geometric information about a shaft from a shaft region of the basket from the captured medical image, and

the processor infers the image-based distance from the estimated geometric information on the basis of a machine learning model.

4. The system of claim 3, wherein the geometric information about the shaft includes at least one of an area occupied by the shaft region of the basket on the medical image, a diameter of an estimated circle corresponding to a distal end portion of the shaft, a radius of the estimated circle corresponding to the distal end portion of the shaft, a length from a boundary of the medical image to the distal end portion of the shaft, a length from a center of the medical image to the distal end portion of the shaft, and an inclination of the shaft.

5. The system of claim 2, wherein the processor infers the image-based distance by inputting the medical image into another machine learning model.

6. The system of claim 2, wherein the processor determines the estimated distance by applying a bias compensation value determined on the basis of the image-based distance and the encoder-based distance to the encoder-based distance.

7. The system of claim 6, wherein the bias compensation value has a value that converges over time after calculation of the estimated distance is started.

8. The system of claim 1, wherein the processor reduces the size of the guidance graphic representation when the estimated distance increases, and

the processor increases the size of the guidance graphic representation when the estimated distance decreases.

9. The system of claim 1, wherein when the medical tool is the laser fiber, the processor senses a moment when a distal end portion of the laser fiber makes contact with a calculus,

the processor captures and stores a target image at a time point where the distal end portion of the laser fiber makes contact with the calculus, and

the processor outputs the medical image to a display of the master device simultaneously with the target image.

10. The system of claim 9, wherein the processor receives an input from a user to select a point on the target image and, in response thereto, outputs the guidance graphic representation on the target image on the basis of the selected point.

11. The system of claim 9, wherein the processor detects a calculus region on the target image and outputs the guidance graphic representation on the target image on the basis of a reference point in the detected calculus region.

12. The system of claim 1, wherein the guidance graphic representation is expressed as one of circular, oval, square, polygonal, and closed curve shapes.

13. The system of claim 1, wherein the size of the guidance graphic representation is defined on the basis of a size of a sheath of the basket of the medical tool.

**14.** The system of claim 1, wherein a boundary of the guidance graphic representation has a thickness, wherein the processor reduces the thickness of the boundary of the guidance graphic representation when reliability of the estimated distance increases over time.

**15.** A method of providing guidance by a processor, the method comprising:

obtaining a medical image captured by including a medical tool at a distal end portion of a guide tube inserted into a ureter;

inferring an image-based distance between the medical tool and a camera at the distal end portion of the guide tube from the captured medical image;

obtaining information about an encoder of a driving unit that moves a wire within the guide tube;

estimating an encoder-based distance between the medical tool and the camera at the distal end portion of the guide tube from the information about the encoder;

determining an estimated distance between the medical tool and the camera at the distal end portion of the guide tube on the basis of the image-based distance and the encoder-based distance; and

displaying a guidance graphic representation of a size determined on the basis of the estimated distance by overlaying the guidance graphic representation on the medical image.

**16.** The method of claim 15, wherein the inferring of the image-based distance includes: when the medical tool is a basket or laser fiber, estimating geometric information about a shaft from a shaft region of the medical tool from the captured medical image; and

inferring the image-based distance from the estimated geometric information on the basis of a machine learning model.

**17.** The method of claim 15, wherein the determining of the estimated distance includes:

determining the estimated distance by applying a bias compensation value determined on the basis of the image-based distance and the encoder-based distance to the encoder-based distance,

wherein the bias compensation value has a value that converges over time after calculation of the estimated distance is started.

**18.** The method of claim 15, wherein the displaying of the guidance graphic representation by overlaying the guidance graphic representation on the medical image further includes:

adjusting the size of the guidance graphic representation on the basis of a changing estimated distance between the medical tool and the camera at the distal end portion of the guide tube,

wherein the size of the guidance graphic representation decreases as the estimated distance increases and increases as the estimated distance decreases.

**19.** The method of claim 15, further comprising:

changing a position of the guidance graphic representation on the medical image in response to an external input of a user by including the medical tool at the distal end portion of the guide tube inserted into the ureter.

**20.** A use method of the system of claim 1, the use method comprising:

displaying a guidance circle according to a distance of a medical tool, i.e., a basket or laser fiber and allowing a user to bring the medical tool into contact with a calculus and perform size comparison.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

estimated distance[mm]

[FIG. 11]

[FIG. 12]

[FIG. 13]

131 132 133

[FIG. 14]

141 142

[FIG. 15]

[FIG. 16]

[FIG. 17]

```
                    ┌─────────────┐
                    │    start    │
                    └─────────────┘
                           │                    ⌐171
                           ▼
        ┌──────────────────────────────────────┐
        │       obtaining medical image        │
        └──────────────────────────────────────┘
                           │                    ⌐172
                           ▼
        ┌──────────────────────────────────────┐
        │     inferring image-based distance    │
        └──────────────────────────────────────┘
                           │                    ⌐173
                           ▼
        ┌──────────────────────────────────────┐
        │   obtaining information about encoder  │
        └──────────────────────────────────────┘
                           │                    ⌐174
                           ▼
        ┌──────────────────────────────────────┐
        │    estimating encoder-based distance   │
        └──────────────────────────────────────┘
                           │                    ⌐175
                           ▼
        ┌──────────────────────────────────────┐
        │     determining estimated distance     │
        └──────────────────────────────────────┘
                           │                    ⌐176
                           ▼
        ┌──────────────────────────────────────┐
        │       displaying guidance graphic      │
        │            representation              │
        └──────────────────────────────────────┘
                           │
                           ▼
                    ┌─────────────┐
                    │     end     │
                    └─────────────┘
```

**INTERNATIONAL SEARCH REPORT**

| International application No. |
|---|
| **PCT/KR2023/010044** |

| | |
|---|---|
| **A.** | **CLASSIFICATION OF SUBJECT MATTER** |
| | **A61B 17/221**(2006.01)i; **A61B 34/30**(2016.01)i; **A61B 17/22**(2006.01)i; **A61B 17/00**(2006.01)i |

According to International Patent Classification (IPC) or to both national classification and IPC

| | |
|---|---|
| **B.** | **FIELDS SEARCHED** |

Minimum documentation searched (classification system followed by classification symbols)

A61B 17/221(2006.01); A61B 18/14(2006.01); A61B 34/00(2016.01); A61B 34/10(2016.01); A61B 8/12(2006.01); A61B 90/00(2016.01); G06N 20/00(2019.01); G16H 30/40(2018.01); G16H 50/20(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 결석(stone), 크기(size), 카메라(camera), 영상(image), 바스켓(basket), 레이저(laser), 가이던스 그래픽(guidance graphics), 오버레이(overlay)

| | |
|---|---|
| **C.** | **DOCUMENTS CONSIDERED TO BE RELEVANT** |

| Category* | Citation of document, with indication, where appropriate, of the relevant passages | Relevant to claim No. |
|---|---|---|
| Y | KR 10-2021-0066560 A (EASYENDO SURGICAL, INC.) 07 June 2021 (2021-06-07) See paragraph [0042]; and claims 1 and 7. | 1-5,8,12-16,18-20 |
| A | | 6,7,9-11,17 |
| Y | JP 2011-500164 A (GYNESONICS, INC.) 06 January 2011 (2011-01-06) See claims 1 and 13. | 1-5,8,12-16,18-20 |
| A | KR 10-2209086 B1 (STARLABS CORP.) 28 January 2021 (2021-01-28) See claims 1 and 3. | 1-20 |
| A | KR 10-2021-0101640 A (UNIVERSITY OF ULSAN FOUNDATION FOR INDUSTRY COOPERATION et al.) 19 August 2021 (2021-08-19) See entire document | 1-20 |

☑ Further documents are listed in the continuation of Box C.   ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 October 2023** | **16 October 2023** |

| Name and mailing address of the ISA/KR | Authorized officer |
|---|---|
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

## INTERNATIONAL SEARCH REPORT

| International application No. |
| --- |
| **PCT/KR2023/010044** |

**C.     DOCUMENTS CONSIDERED TO BE RELEVANT**

| Category* | Citation of document, with indication, where appropriate, of the relevant passages | Relevant to claim No. |
| --- | --- | --- |
| A | KR 10-2021-0069542 A (FOUNDATION OF SOONGSIL UNIVERSITY-INDUSTRY COOPERATION) 11 June 2021 (2021-06-11) See entire document | 1-20 |

Form PCT/ISA/210 (second sheet) (July 2022)

## INTERNATIONAL SEARCH REPORT
### Information on patent family members

International application No.

**PCT/KR2023/010044**

| Patent document cited in search report | | | Publication date (day/month/year) | Patent family member(s) | | | Publication date (day/month/year) |
|---|---|---|---|---|---|---|---|
| KR | 10-2021-0066560 | A | 07 June 2021 | KR | 10-2386660 | B1 | 15 April 2022 |
| | | | | WO | 2021-107748 | A2 | 03 June 2021 |
| | | | | WO | 2021-107748 | A3 | 22 July 2021 |
| JP | 2011-500164 | A | 06 January 2011 | CA | 2702353 | A1 | 16 April 2009 |
| | | | | CA | 2702353 | C | 21 June 2016 |
| | | | | EP | 2209423 | A1 | 28 July 2010 |
| | | | | EP | 2209423 | A4 | 04 May 2016 |
| | | | | EP | 2209423 | B1 | 04 July 2018 |
| | | | | EP | 3420916 | A2 | 02 January 2019 |
| | | | | EP | 3420916 | A3 | 06 March 2019 |
| | | | | EP | 3420916 | B1 | 13 May 2020 |
| | | | | EP | 3733106 | A1 | 04 November 2020 |
| | | | | ES | 2685448 | T3 | 09 October 2018 |
| | | | | ES | 2812341 | T3 | 16 March 2021 |
| | | | | JP | 2014-050748 | A | 20 March 2014 |
| | | | | JP | 2016-025933 | A | 12 February 2016 |
| | | | | JP | 2018-047295 | A | 29 March 2018 |
| | | | | JP | 2019-072535 | A | 16 May 2019 |
| | | | | JP | 2022-033982 | A | 02 March 2022 |
| | | | | JP | 5964010 | B2 | 03 August 2016 |
| | | | | JP | 6473404 | B2 | 20 February 2019 |
| | | | | JP | 6674432 | B2 | 01 April 2020 |
| | | | | US | 11096760 | B2 | 24 August 2021 |
| | | | | US | 11096761 | B2 | 24 August 2021 |
| | | | | US | 2009-0099544 | A1 | 16 April 2009 |
| | | | | US | 2012-0078134 | A1 | 29 March 2012 |
| | | | | US | 2012-0316440 | A1 | 13 December 2012 |
| | | | | US | 2017-0245838 | A1 | 31 August 2017 |
| | | | | US | 2017-0245891 | A1 | 31 August 2017 |
| | | | | US | 2020-0229892 | A1 | 23 July 2020 |
| | | | | US | 2021-0000565 | A1 | 07 January 2021 |
| | | | | US | 2021-0038341 | A1 | 11 February 2021 |
| | | | | US | 2021-0338365 | A1 | 04 November 2021 |
| | | | | US | 2022-0265381 | A1 | 25 August 2022 |
| | | | | US | 8088072 | B2 | 03 January 2012 |
| | | | | US | 8262577 | B2 | 11 September 2012 |
| | | | | WO | 2009-049082 | A1 | 16 April 2009 |
| KR | 10-2209086 | B1 | 28 January 2021 | US | 11521318 | B2 | 06 December 2022 |
| | | | | US | 2022-0067936 | A1 | 03 March 2022 |
| KR | 10-2021-0101640 | A | 19 August 2021 | KR | 10-2356341 | B1 | 28 January 2022 |
| | | | | WO | 2021-162355 | A1 | 19 August 2021 |
| KR | 10-2021-0069542 | A | 11 June 2021 | | None | | |

Form PCT/ISA/210 (patent family annex) (July 2022)